(19) Europäisches Patentamt
European Patent Office
Office européen des brevets

(11) **EP 0 892 617 B1**

(12) **EUROPÄISCHE PATENTSCHRIFT**

(45) Veröffentlichungstag und Bekanntmachung des Hinweises auf die Patenterteilung:
**19.09.2001 Patentblatt 2001/38**

(21) Anmeldenummer: **97906973.9**

(22) Anmeldetag: **26.03.1997**

(51) Int Cl.$^7$: **A61B 5/00**, A61B 5/024

(86) Internationale Anmeldenummer:
**PCT/CH97/00125**

(87) Internationale Veröffentlichungsnummer:
**WO 97/36538 (09.10.1997 Gazette 1997/43)**

(54) **ERKENNUNG VON STÖRSIGNALEN BEI DER PULSOXYMETRISCHEN MESSUNG**

DETECTION OF PARASITIC SIGNALS DURING PULSOXYMETRIC MEASUREMENT

DETECTION DE SIGNAUX PARASITES LORS DE MESURES PULSOXYMETRIQUES

(84) Benannte Vertragsstaaten:
**CH DE ES FR GB IT LI**

(30) Priorität: **01.04.1996 CH 84696**

(43) Veröffentlichungstag der Anmeldung:
**27.01.1999 Patentblatt 1999/04**

(73) Patentinhaber: **Linde Medical Sensors AG**
**4051 Basel (CH)**

(72) Erfinder:
• **SCHÖLLERMANN, Hans**
**VERSTORBEN (CH)**

• **EBERHARD, Patrick**
**CH-4123 Allschwil (CH)**

(74) Vertreter: **Bollhalder, Renato et al**
**Braun Héritier Eschmann AG**
**Patentanwälte VSP**
**Holbeinstrasse 36-38**
**Postfach 160**
**4003 Basel (CH)**

(56) Entgegenhaltungen:
WO-A-94/03102          DE-A- 3 723 881
US-A- 3 152 587

**Beschreibung**

Die Erfindung betrifft ein Verfahren zur Erkennung von durch Bewegungen eines Patienten oder dessen Umgebung verursachten Störsignalen bei der pulsoxymetrischen Messung der arteriellen Sauerstoffsättigung.

Es ist bekannt, die Sauerstoffsättigung des Hämoglobins im arteriellen Blut (arterielle Sauerstoffsättigung) mittels einer nichtinvasiven Methode, die als Pulsoxymetrie bezeichnet wird, zu messen. Diese Methode wird zur Überwachung von Patienten z.B. während Anästhesie und Intensivpflege eingesetzt. Das Prinzip der Messung beruht auf dem Unterschied zwischen den Lichtabsorptionsfähigkeiten von Hämoglobin in seiner mit Sauerstoff gesättigten und seiner reduzierten Form. Der Absorptionskoeffizient von Blut ist bei rotem Licht stark vom Sauerstoffgehalt abhängig und bei Licht im nahen Infrarotbereich davon nahezu unabhängig. Durch Messung des Intensitätsverhältnisses des absorbierten Lichtes beider Wellenlängen kann die arterielle Sauerstoffsättigung ermittelt werden.

In der Pulsoxymetrie werden als Lichtquellen in der Regel zwei dicht nebeneinanderliegende lichtemittierende Dioden (LED) mit Wellenlängen von etwa 660 nm (rot) und etwa 890 nm (infrarot) verwendet. Das von den beiden LED ausgestrahlte Licht wird in einen gut durchbluteten Körperteil (z.B. die Fingerbeere) geleitet und dort gestreut und teilweise absorbiert. Das austretende Licht wird mit einer Fotodiode gemessen, die in der Regel den LED gegenüberliegend angeordnet ist. Die LED und die Fotodiode sind üblicherweise in einer Baueinheit integriert, die als pulsoxymetrischer Sensor bezeichnet wird. Die separate Messung des roten und infraroten Lichtes mit nur einer Fotodiode wird durch Verwendung von alternierenden Lichtimpulsen der beiden Wellenlängen ermöglicht, die getrennt messtechnisch erfasst und ausgewertet werden.

Das von der Fotodiode gemessene Licht beider Wellenlängen besteht aus einer stationären und einer zeitabhängigen Komponente. Die stationäre Komponente ist im wesentlichen durch Absorption durch Knochen, Gewebe, Haut und nicht pulsierendes Blut bestimmt. Die zeitabhängige Komponente wird durch Absorptionsänderungen im Messobjekt verursacht, die im Idealfall nur durch das pulsförmig einströmende arterielle Blut hervorgerufen werden. Für die Ermittlung der arteriellen Sauerstoffsättigung ($SaO_2$) werden die stationären Komponenten ($DC_R$, $DC_{IR}$) und die zeitabhängigen Komponenten ($AC_R$, $AC_{IR}$) der gemessenen roten (R) und infraroten (IR) Lichtintensitäten verwertet. Üblicherweise wird die arterielle Sauerstoffsättigung über die Beziehung

$$SaO_2 = f\left(\frac{AC_R}{DC_R} : \frac{AC_{IR}}{DC_{IR}}\right) \qquad (1)$$

ermittelt, wobei f eine empirisch bestimmte Funktion darstellt.

Ein noch nicht zufriedenstellend gelöstes Problem bei der pulsoxymetrischen Messung besteht darin, dass Störungen der Messsignale, die durch Bewegungen des Patienten oder dessen Umgebung verursacht werden, nicht vollständig eliminiert werden können. Derartige Störungen sind besonders dann kritisch, wenn sie periodisch auftreten, weil sie in diesem Fall unter bestimmten Bedingungen zu falschen Messergebnissen führen können. Da die Frequenzverteilung von Bewegungsartifakten diejenige des physiologischen Signals überlappen kann, sind konventionelle Bandpassfilter oder selektive Filter nicht dazu geeignet, Bewegungsartifakte von dem physiologischen Signal zuverlässig zu trennen. Auch adaptive Filtertechniken, wie z.B. die Methode der adaptiven Störfrequenzunterdrückung, können nicht direkt auf die Pulsoxymetrie angewendet werden, da diese voraussetzen, dass entweder die Störfrequenzen oder die physiologischen Signale voraussehbare Frequenzcharakteristiken aufweisen. Diese Voraussetzung trifft weder für Bewegungsartifakte noch für die Pulsfrequenz zu. Letztere kann insbesondere bei Patienten mit kardiovaskulären Krankheiten eine hohe Variabilität aufweisen.

Grundsätzlich muss festgehalten werden, dass infolge der Natur des Problemes jeder Lösung, die allein auf einer Verbesserung der Signalverarbeitung beruht, Grenzen gesetzt sind. Diese Grenzen sind dadurch bedingt, dass Störungen durch Bewegungsartifakte nicht vollständig ausgeschaltet werden können, da sie nicht immer als solche erkannt werden. Primär muss deshalb eine Lösung über den Weg einer differenzierten Signalgewinnung gesucht werden, die eine Trennung der Störsignale von den physiologischen Signalen ermöglicht. In dieser Richtung sind bereits verschiedene Lösungswege vorgeschlagen worden.

In der WO-A-94/03102 ist eine optische Überwachungsvorrichtung beschrieben, die umfasst:

a) einen Sensor mit einem Senderteil, der drei lichtemittierende Dioden aufweist, die Licht unterschiedlicher Wellenlänge emittieren, und mit einem Empfängerteil zur Lichtintensitätsmessung, der drei Fotodetektoren aufweist, sowie
b) einen Steuer- und Auswerteteil.
Zur Unterdrückung der durch Bewegungen eines Patienten oder dessen Umgebung verursachten Störsignale wird vorgeschlagen, die an den Fotodetektoren erzeugten Signale auf gleiche Niveaus ihrer DC-Anteile zu normieren.

Ausgehend von der Annahme, dass die Amplituden der Störkomponenten dieser normierten Signale gleich gross sind,-werden dann zur Beseitigung der Störkomponenten die Differenzen der normierten Signale gebildet. In der Praxis sind aber die Amplituden der Störkomponenten der normierten Signale unterschiedlich, wie auch auf Seite 6, Zeilen 4 bis 6 dieser Publikation erwähnt wird. Eine vollständige Störsignalunterdrückung erfolgt somit nicht.

In der US-A-4,802,486 ist z.B. eine Methode beschrieben, die darauf beruht, bestimmte aus dem EKG des Patienten abgeleitete Messsignale zur Identifizierung der arteriellen Pulsationen zu verwenden. Nicht als solche identifizierte Signale (d.h. Störsignale) können somit unterdrückt werden. Diese als EKG-synchronisierte Pulsoxymetrie bezeichnete Methode hat den Nachteil, dass Störsignale, die gleichzeitig mit einem Pulssignal auftreten, nicht erfasst werden. Ausserdem wird die simultane Messung des EKG vorausgesetzt. Dieses ist jedoch nicht immer verfügbar.

In der US-A-5,226,417 wird vorgeschlagen, in den pulsoxymetrischen Sensor einen Messwertaufnehmer einzubauen, der Bewegungen am Ort der pulsoxymetrischen Messstelle detektiert. Als Beispiele für derartige Messwertaufnehmer werden piezoelektrische Filme, Beschleunigungstransducer und Dehnungsmessstreifen genannt. Eine solche Lösung bedingt jedoch einen beträchtlichen Aufwand in der Herstellung des Sensors, was zu einer wesentlichen Verteuerung des oft als Einwegartikel konzipierten Produktes führt. Zudem ist wegen der extrem hohen Anforderungen an die Empfindlichkeit der Bewegungsdetektierung ein beträchtlicher Aufwand für die Signalverarbeitung erforderlich.

Eine ähnliche Idee ist in der US-A-5,025,791 beschrieben. Hierin wird vorgeschlagen, in den pulsoxymetrischen Sensor einen speziell zu diesem Zweck konstruierten Bewegungsdetektor einzubauen, der auf einer elektromechanischen oder einer magnetischen Messmethode oder einer Kombination dieser beiden Methoden beruht. Gegen ein solches Konzept sprechen die bereits oben angeführten Einwände.

Eine andere Lösung ist in der WO-A-91/18550 vorgeschlagen. Darin wird eine Anordnung beschrieben, die zur Messung der Pulsfrequenz vorgesehen ist, deren Konzept jedoch auch auf die Pulsoxymetrie übertragen werden könnte. In einem Sensor, der an der Stirn einer Person appliziert wird, sind eine im infraroten und eine im gelben Frequenzbereich emittierende LED sowie zwei Fotodioden eingebaut. Das in das Stirngewebe eingestrahlte Licht wird dort zurückgestreut und mit den beiden Fotodioden gemessen. Das mit dem infraroten Licht erzeugte Signal enthält Komponenten, die sowohl durch das pulsierende arterielle Blut als auch durch Bewegungen verursacht werden. Dagegen ist das mit dem gelben Licht erzeugte Signal weitgehend unabhängig von Blutpulsationen und enthält nur die durch Bewegungen verursachten Komponenten. Dies lässt sich dadurch erklären, dass infrarotes Licht tief in das gut durchblutete Stirngewebe einzudringen vermag, während gelbes Licht eine wesentlich kürzere Eindringtiefe hat und deshalb nur Vorgänge in der Nähe der Oberfläche der Stirnhaut, d.h. in einem schwach durchbluteten Bereich, erfasst. Die beiden Signale können nun durch bekannte Verfahren analysiert und die durch Bewegungen verursachten Anteile des infraroten Signals entfernt werden. Bei einer Übertragung dieses Konzeptes auf die Pulsoxymetrie ist zu bedenken, dass die Stirn aus vorwiegend praktischen aber auch aus physiologischen Gründen als Messstelle schlecht geeignet ist. Die für pulsoxymetrische Messungen am häufigsten verwendete Messstelle ist der Finger. Oft werden Messungen auch am Ohrläppchen und an der Zehe durchgeführt. Diesen drei Messstellen ist gemeinsam, dass auch die oberflächennahen Gewebeteile gut durchblutet sind. Somit ist dort eine Trennung der durch Pulsationen und durch Bewegungen bedingten Signalkomponenten durch Verwendung von Licht verschiedener Eindringtiefen nicht ohne weiteres möglich.

Der Erfindung liegt die Aufgabe zugrunde, eine verbesserte Methode zur differenzierten Signalgewinnung für pulsoxymetrische Messungen zu schaffen, die eine Erkennung der durch Bewegungen bedingten Störsignale ermöglicht, und die nicht die Nachteile und Begrenzungen der bekannten Methoden aufweist.

Diese Aufgabe wird durch das erfindungsgemässe Verfahren zur Erkennung von Störsignalen, wie es im Patentanspruch 1 definiert ist, gelöst. Bevorzugte Ausführungsvarianten ergeben sich aus den abhängigen Patentansprüchen.

Als pulsoxymetrischer Sensor wird vorzugsweise eine Vorrichtung mit einem Senderteil, der zwei lichtemittierende Dioden aufweist, die Licht unterschiedlicher Wellenlänge emittieren, und mit einem Empfängerteil zur Lichtintensitätsmessung, der mindestens zwei Fotodioden umfasst, verwendet. Die zwei oder mindestens zwei der Fotodioden sind wie bei den bisher bekannten pulsoxymetrischen Sensoren den roten und infraroten LED gegenüberliegend, aber bezüglich der Längsmittellinie der dem Senderteil zugewandten Auflagefläche des Empfängerteils auf verschiedene Seiten versetzt angeordnet.

Eine Ausführungsform eines solchen Sensors hat die Form einer Klammer, die zur Befestigung an einem Finger geeignet ist. Im einen Klammerschenkel sind die roten und infraroten LED eingebaut, deren Licht in die Fingerbeere eingestrahlt wird. Ihnen gegenüberliegend im anderen Klammerschenkel befinden sich zwei nebeneinanderliegende Fotodioden. Das von den LED ausgestrahlte Licht wird in der Fingerbeere mehrfach gestreut, teilweise absorbiert und teilweise ausgestreut. Ein Teil des ausgestreuten Lichtes gelangt zu den Fotodioden. Das von den Fotodioden empfangene Licht besteht wie anfangs beschrieben aus einer stationären und einer zeitabhängigen, durch Blutpulsationen verursachten Komponente. Im Fall von Bewegungen des Patienten oder dessen Umgebung, die sich auf die Messstelle übertragen, tritt hierdurch bedingt eine weitere zeitabhängige Komponente hinzu. Die an den beiden Fotodioden 1 und 2 gemessenen Signale $S_1$ und $S_2$ (Lichtintensitäten) setzen sich demnach wie folgt zusammen:

$$S_1 = DC_1 + AC_{P_1} + AC_{B_1}$$

$$S_2 = DC_2 + AC_{P_2} + AC_{B_2} \tag{2}$$

Hierbei sind $DC_1$ und $DC_2$ die stationären Signalkomponenten, d.h. diejenigen Lichtintensitäten, die in völliger Abwesenheit von Blutpulsationen und Bewegungsartifakten gemessen würden. $AC_{P_1}$ bzw. $AC_{P_2}$ sind die durch Blutpulsationen und $AC_{B_1}$ bzw. $AC_{B_2}$ die durch Bewegungen verursachten Signalkomponenten.

Die an den beiden Fotodioden gemessenen Signale $S_1$ und $S_2$ sind in Abwesenheit von Bewegungsartifakten sehr ähnlich. Im Fall von Bewegungen treten jedoch sehr deutliche Unterschiede zwischen ihnen auf. Um diese Unterschiede zu erfassen, werden $S_1$ und $S_2$ zunächst auf eine annähernd gleiche Amplitude normiert, was zweckmässigerweise mittels Division durch die jeweiligen DC-Werte erfolgt:

$$S_{1n} = \frac{S_1}{DC_1} = 1 + \frac{AC_{P_1}}{DC_1} + \frac{AC_{B_1}}{DC_1}$$

$$S_{2n} = \frac{S_2}{DC_2} = 1 + \frac{AC_{P_2}}{DC_2} + \frac{AC_{B_2}}{DC_2} \tag{3}$$

Die somit erhaltenen normierten Signale $S_{1n}$ und $S_{2n}$ werden anschliessend voneinander subtrahiert, und es ergibt sich das Differenzsignal $\Delta S_n$:

$$\Delta S_n = S_{1n} - S_{2n} = \left(\frac{AC_{P_1}}{DC_1} - \frac{AC_{P_2}}{DC_2}\right) + \left(\frac{AC_{B_1}}{DC_1} - \frac{AC_{B_2}}{DC_2}\right)$$

$$bzw. \tag{4}$$

$$\Delta S_n = \Delta S_{nP} + \Delta S_{nB}$$

Hierbei sind $\Delta S_{np}$ die durch Pulsationen und $\Delta S_{nB}$ die durch Bewegungen bedingten Komponenten des Differenzsignals. Messungen, die später ausführlich beschrieben werden, zeigen, dass in Abwesenheit von Bewegungen ($AC_{B_1} = AC_{B_2} = 0$) das mit rotem und auch das mit infrarotem Licht ermittelte Differenzsignal $\Delta S_n$ nahezu gleich Null ist. Dies bedeutet, dass die durch Blutpulsationen verursachten Änderungen im Messobjekt an den beiden benachbarten Fotodioden fast identische Signale erzeugen. Dies lässt sich dadurch erklären, dass das rote und infrarote Licht im Messobjekt ausgiebig gestreut wird und deshalb das Gewebe der Fingerbeere gleichmässig ausgeleuchtet wird. Die durch Blutpulsationen verursachten Änderungen der optischen Eigenschaften des Messobjekts wirken sich deshalb symmetrisch auf beide Messstellen aus.

Im Gegensatz hierzu haben die durch Bewegungen verursachten Änderungen der optischen Eigenschaften des Messobjekts andere Auswirkungen. In diesem Fall lassen sich deutliche Schwankungen des Differenzsignals $\Delta S_n$ feststellen, die zeitlich synchron mit den Bewegungen verlaufen. Das Differenzsignal $\Delta S_n$ erweist sich somit als ein äusserst empfindlicher Indikator zur Detektierung von Bewegungsartifakten verschiedener Art, d.h. unabhängig davon, ob diese durch den Patienten selbst, durch äussere Einwirkungen auf den Sensor und das Kabel oder durch andere Fremdeinflüsse hervorgerufen werden. Insbesondere lassen sich periodisch stattfindende Bewegungsartifakte erkennen, auch wenn diese mit einer Frequenz auftreten, die in der Nähe der Pulsfrequenz liegt oder mit dieser sogar identisch ist. Eine Erklärung für dieses unerwartete Ergebnis muss darin gesucht werden, dass Bewegungen (im Gegensatz zu Blutpulsationen) eine nicht gleichmässig verteilte Änderung der optischen Eigenschaften des Messobjekts zur Folge haben. Bei Bewegungen wird das Gewebe der Fingerbeere in Bezug auf die Positionen der LED und der Fotodioden unsymmetrisch verschoben, was sich in einer ungleichen Änderung der an den beiden benachbarten Fotodioden gemessenen Signale auswirkt.

Die mit dem erfindungsgemässen Verfahren erzielten Vorteile bestehen darin, dass erstmals eine technisch einfach zu realisierende, preisgünstige, empfindliche und zuverlässige Methode zur Erkennung von Bewegungsartifakten während pulsoxymetrischer Messungen zur Verfügung steht. Die Konstruktion eines pulsoxymetrischen Sensors mit zwei oder mehr Fotodioden unterscheidet sich nur unwesentlich von den bisher gebräuchlichen Konstruktionen, da die gleichen optischen Bauelemente verwendet werden. Es ist nicht notwendig, einen separaten Messwertaufnehmer zur Detektion von Bewegungen zu verwenden, wie in den oben angeführten US-A-5,226,417 und US-A-5,025,791 vorgeschlagen wird. Die Signalverarbeitung ist äusserst einfach und kann auf den zur Zeit in der Pulsoxymetrie verwendeten Methoden aufbauen. Das Verfahren ist an jeder der für Pulsoxymetrie gebräuchlichen Messstellen anwendbar. Auch bestehen keine einschränkenden Bedingungen bezüglich der gleichzeitigen Verfügbarkeit eines zweiten Messparameters wie bei der EKG-synchronisierten Pulsoxymetrie (s. US-A-4,802,486). Ein weiterer Vorteil des Verfahrens besteht darin, dass mit der Amplitude des Differenzsignals $\Delta S_n$ ein Mass für die Grösse des Störsignals zur Verfügung steht und die Störung somit auf einfache Art quantifiziert werden kann.

Die Erkennung von Bewegungsartifakten mit Hilfe des erfindungsgemässen Verfahrens ermöglicht es, verschiedene geeignete Massnahmen zu treffen. So können die aus dem Differenzsignal $\Delta S_n$ ermittelten Störsignale mit Hilfe von bekannten Methoden der Analog- oder Digitalelektronik, auf die hier nicht näher eingegangen wird, analysiert, aufbereitet und von dem gemessenen Signal getrennt werden. Das hierdurch erhaltene bereinigte physiologische Signal erlaubt eine genauere und zuverlässigere Bestimmung der arteriellen Sauerstoffsättigung. Ferner kann bei Auftreten von Bewegungsartifakten eine Alarmmeldung ausgelöst werden, die den Benutzer des Pulsoxymeters dazu veranlasst, die Ursachen für die Störungen zu beseitigen. Auch besteht die Möglichkeit, die Amplitude des Störsignals mit der des physiologischen Signals zu vergleichen und ab einem bestimmten Verhältnis dieser beiden Werte eine Alarmmeldung auszulösen oder das Messresultat nicht mehr anzuzeigen. Dies kann z. B. dann notwendig werden, wenn während schwacher physiologischer Signale derartig starke Störungen auftreten, dass die Anforderungen an die Messgenauigkeit für die arterielle Sauerstoffsättigung nicht mehr erfüllt werden können.

Nachfolgend wird ein bevorzugtes Ausführungsbeispiel der Erfindung anhand der beiliegenden Zeichnungen beschrieben.

Es zeigen:

Figuren 1A-1C   schematische Ansichten eines pulsoxymetrischen Sensors für Messungen am Finger;
Figur 2   ein Funktionsschema der erfindungswesentlichen Schaltungsteile eines Pulsoxymeters und
Figur 3   ein Beispiel von erfindungsgemäss bearbeiteten Lichtintensitäts-Verlaufskurven.

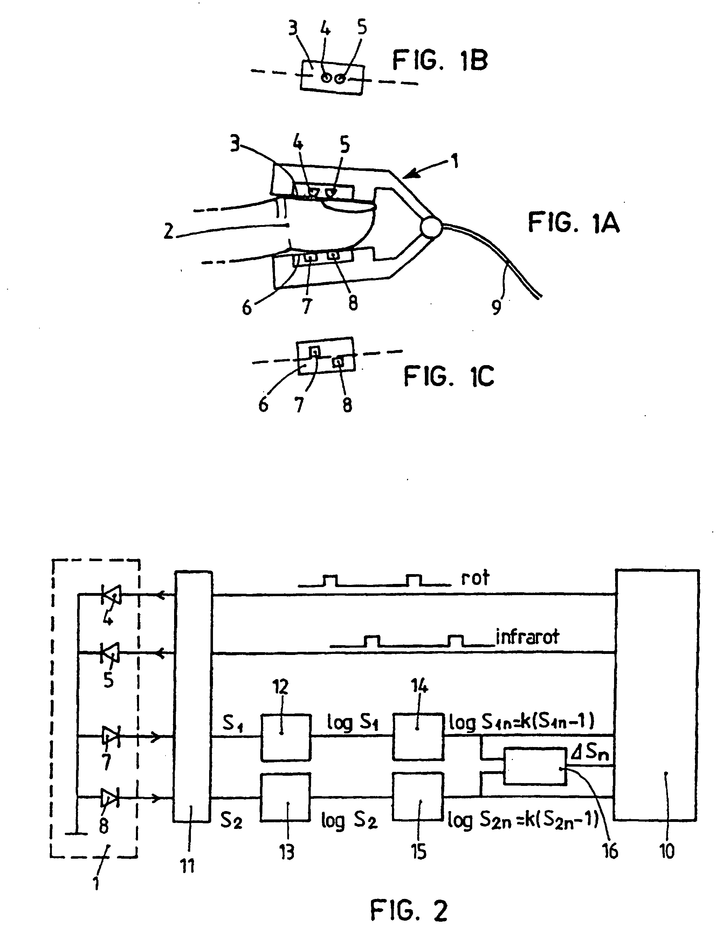

Der in Figur 1A gezeigte Sensor 1 hat die Form einer Klammer, die an einem Finger 2 befestigt ist. In der Auflagefläche 3 des oberen Klammerschenkels befinden sich eine rote 4 und eine infrarote 5 LED, deren Licht in die Fingerbeere einstrahlt. Die Auflagefäche 6 des gegenüberliegenden Klammerschenkels enthält zwei Fotodioden 7 und 8 zur Messung des durchgelassenen Lichtes. Die vier Komponenten 4, 5, 7 und 8 sind über ein Kabel 9 mit dem Steuer- und Auswerteteil des Pulsoxymeters verbunden. Die Figuren 1B und 1C zeigen jeweils eine Ansicht auf die Auflageflächen 3 und 6, um die Positionen der LED und der Fotodioden zu verdeutlichen. Die LED 4 und 5 sind in einem möglichst geringen Abstand (ca. 1 bis 2 mm) längs der Mittellinie der Auflagefläche 3, die etwa der Mittellinie des Fingers entspricht, eingebaut. Dagegen sind die Fotodioden 7 und 8 schräg versetzt zur Mittellinie der Auflagefläche 6 angeordnet. Der Abstand zwischen den Fotodioden kann je nach ihrer Grösse und der Grösse des Sensors etwa 1 bis 10 mm betragen. Mit dieser geometrischen Anordnung der Fotodioden wird folgendes bezweckt: Wie oben beschrieben besteht eine Idee darin, die durch Bewegungen bedingten Änderungen der optischen Eigenschaften des Messobjekts auf Grund ihrer ungleichen Auswirkungen auf zwei benachbarte Messstellen zu erkennen. Derartige Änderungen können sich je nach Art der Bewegung mehr in Richtung der Fingermittellinie oder senkrecht dazu auswirken. Mit Hilfe der diagonal zur Fingermittellinie versetzten Anordnung der Fotodioden können somit Änderungen in beiden Richtungen erfasst werden.

Es versteht sich, dass an Stelle des hier beschriebenen klammerförmigen Fingersensors auch andere in der Pulsoxymetrie gebräuchliche Sensortypen, z. B. Sensoren mit flexiblen Auflageflächen, verwendet werden können. Auch kommen ausser dem Finger alle anderen für pulsoxymetrische Messungen üblicherweise verwendeten Körperteile (z.B. Ohren, Zehen) in Frage. Ferner ist das Konzept der Erfindung nicht notwendigerweise auf die Verwendung von nur zwei Fotodioden und auf die hier beschriebene geometrische Anordnung beschränkt. Es ist offensichtlich, dass die Empfindlichkeit der Erkennung von Bewegungsartifakten durch Verwendung von drei oder mehr Fotodioden erhöht werden kann, wobei deren geometrische Anordnung je nach Sensortyp, Messstelle und Anzahl der verwendeten Fotodioden im Einzelfall optimal gestaltet werden muss.

Figur 2 zeigt ein vereinfachtes Funktionsschema derjenigen Schaltungsteile, die zur Ermittlung des Differenzsignals $\Delta S_n$ erforderlich sind. Zur Vereinfachung sind alle Schaltungselemente, die nicht unmittelbar zum Verständnis des erfindungsgemässen Verfahrens wichtig sind, nicht separat gezeigt, sondern in der Elektronikeinheit 10 schematisch zusammengefasst. Die roten und infraroten LED 4 und 5 des Sensors 1 werden über die Elektronikeinheit 10 und den Multiplexer 11 so angesteuert, dass alternierende Lichtimpulse beider Wellenlängen erzeugt werden. Die an den Fotodioden 7 und 8 des Sensors 1 erhaltenen Signale $S_1$ und $S_2$ bestehen wie oben beschrieben (siehe Gleichung 2) jeweils aus einer DC-Komponente ($DC_1$, $DC_2$), einer ersten AC-Komponente, die durch Blutpulsationen hervorgerufen wird ($AC_{P1}$, $AC_{P2}$), und einer zweiten AC-Komponente, die durch Bewegungen verursacht wird ($AC_{B1}$, $AC_{B2}$). Jedes dieser Signale ist sowohl für das rote als auch für das infrarote Licht vorhanden, wobei es jedoch in der folgenden Beschreibung keine Rolle spielt, welche der beiden Lichtwellenlängen für die Ermittlung von $\Delta S_n$ verwendet wird. Mittels der Verstärker 12 und 13 werden $S_1$ und $S_2$ zunächst logarithmiert. Anschliessend werden mittels der Hochpassfilter 14 und 15 die DC-Komponenten der logarithmierten Signale entfernt. Mathematisch lässt sich die Hochpassfilterung des logarithmierten Signals $S_1$ wie folgt beschreiben:

$$\log(DC_1 + AC_{P1} + AC_{B1}) - \log DC_1$$

$$= \log(1 + \frac{AC_{P1}}{DC_1} + \frac{AC_{B1}}{DC_1}) = \log S_{1n} \tag{5}$$

$S_{1n}$ ist durch Gleichung 3 definiert. Da in der Regel die AC-Komponenten des Signals wesentlich kleiner sind als der DC-Anteil, d.h., da

$$\frac{AC_{P1} + AC_{B1}}{DC_1} \ll 1 \tag{6}$$

gilt näherungsweise die Beziehung:

$$\log S_{1n} = k \cdot (\frac{AC_{P1}}{DC_1} + \frac{AC_{B1}}{DC_1}) = k \cdot (S_{1n} - 1), \tag{7}$$

wobei k = log e = 0,434.

Für $S_{2n}$ gilt entsprechend:

$$\log S_{2n} = k \cdot (\frac{AC_{P2}}{DC_2} + \frac{AC_{B2}}{DC_2}) = k \cdot (S_{2n} - 1) \tag{8}$$

Mit dem Verstärker 16 wird folgende Differenz gebildet:

$$\log S_{1n} - \log S_{2n} = k \cdot \Delta S_n, \tag{9}$$

Das somit erhaltene Differenzsignal $\Delta S_n$ (Definition siehe Gleichung 4) steht nun zur weiteren Verarbeitung und Auswertung durch die Elektronikeinheit 10 zur Verfügung. Auf die dabei verwendeten Methoden wird hier nicht näher eingegangen, da sie nicht zum Gegenstand der Erfindung gehören.

Es versteht sich, dass an Stelle des hier beschriebenen Schaltungskonzeptes auch andere Schaltkreise verwendet werden können, sofern sie zur Ermittlung des normierten Differenzsignals $\Delta S_n$ gemäss obiger Definition oder einer hierzu äquivalenten Grösse geeignet sind. So kommen z. B. auch Schaltungen in Frage, in denen statt des hier gewählten Weges der Logarithmierung die AC-Komponenten der Signale $S_1$ und $S_2$ direkt durch ihre DC-Anteile dividiert werden und anschliessend die Differenz der so erhaltenen zwei Quotienten gebildet wird. Es ist auch möglich, die Signale $S_1$ und $S_2$ zunächst auf gleiche Niveaus ihrer DC-Anteile zu verstärken und anschliessend die Differenz der auf diese Weise normierten Signale zu bilden.

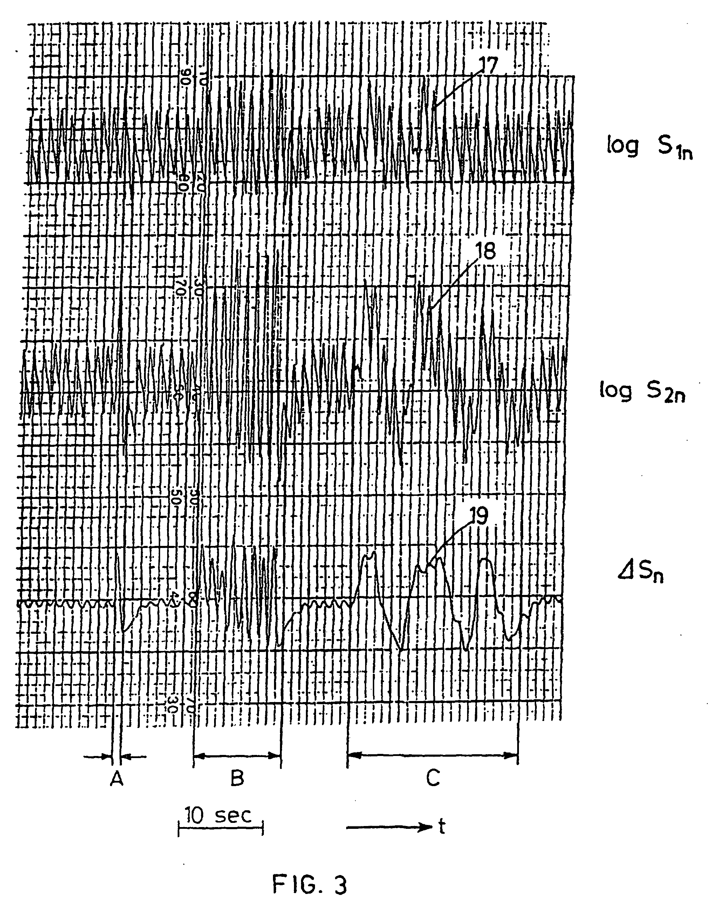

Figur 3 zeigt als Beispiel ein Messresultat, das in Abwesenheit und Anwesenheit von Bewegungsartifakten erhalten wurde. Die oberen beiden Kurven 17 und 18 zeigen die nach den Hochpassfiltern 14 und 15 abgegriffenen Werte von $\log S_{1n} = k(S_{1n}-1)$ und $\log S_{2n} = k(S_{2n}-1)$. Die unterste Kurve 19 zeigt das nach dem Differenzverstärker 16 erhaltene Signal $\Delta S_n$. Zum Zeitpunkt A ereignet sich auf Grund einer Bewegung des Sensors eine einzelne kurze Störung. Während der Zeitintervalle B und C treten periodische Störungen auf, die jeweils durch schnelle (B) und langsame (C) rythmische Bewegungen der Messstelle (Finger) hervorgerufen wurden. Es ist zu erkennen, dass bei Abwesenheit von Bewegungen $\log S_{1n}$ und $\log S_{2n}$ sehr ähnlich verlaufen und $\Delta S_n$ nur geringfügige, pulssynchrone Schwankungen aufweist. Dies ist wie oben erwähnt darauf zurückzuführen, dass die durch Blutpulsationen bedingten Signalkomponenten von $S_{1n}$ und $S_{2n}$ fast gleiche Amplituden haben. Im Fall von Bewegungen sind dagegen starke

Schwankungen von $\Delta S_n$ zu erkennen, die zeitlich synchron zu den Bewegungsabläufen erfolgen. Hierbei ist hervorzuheben, dass die während der Zeit B auftretenden Störsignale etwa die gleiche Frequenz wie die Pulsrate haben und in diesem speziellen Fall für $\log S_{1n}$ und $\log S_{2n}$ Kurvenformen erzeugen, die sich von rein physiologischen Signalen nicht unterscheiden lassen. Störsignale dieser extremen Art können deshalb mit herkömmlichen Methoden der Signalanalyse nicht erkannt werden. Es ist ein besonderer Vorteil der Erfindung, sogar Störungen dieser Art zuverlässig zu detektieren.

**Patentansprüche**

1. Verfahren zur Erkennung von durch Bewegungen eines Patienten oder dessen Umgebung verursachten Störsignalen bei der pulsoxymetrischen Messung der arteriellen Sauerstoffsättigung, bei dem die Intensität des aus dem Messobjekt austretenden Lichtes mit zwei oder mehr Fotodioden (7, 8) gemessen wird, die an diesen Fotodioden (7, 8) erzeugten Signale ($S_1$, $S_2$) auf gleiche Niveaus ihrer DC-Anteile normiert werden und zur Erkennung von bewegungsbedingten Störsignalen die Differenzen ($\Delta S_n$) dieser normierten Signale ($S_{1n}, S_{2n}$) gebildet werden, wobei die Amplituden der bei der Differenzbildung erhaltenen Differenzsignale ($\Delta S_n$) als Mass für die Grösse der bewegungsbedingten Störsignale verwendet werden.

2. Verfahren nach Anspruch 1, **dadurch gekennzeichnet, dass** das Verhältnis zwischen der Amplitude eines Differenzsignals ($\Delta S_n$) und der Amplitude eines normierten Signals ($S_{1n}$, $S_{2n}$) gebildet wird und dieses Verhältnis als Mass für die Relevanz der bewegungsbedingten Störsignale hinsichtlich ihres Einflusses auf die Genauigkeit der pulsoxymetrischen Messung der arteriellen Sauerstoffsättigung verwendet wird.

3. Verfahren nach Anspruch 1 oder 2, **dadurch gekennzeichnet, dass** die Normierung der an den Fotodioden (7, 8) erzeugten Signale ($S_1$, $S_2$) dadurch erfolgt, dass diese in einem ersten Schritt logarithmiert werden und in einem zweiten Schritt die DC-Anteile der logarithmierten Signale mit Hilfe von Hochpassfiltern entfernt werden.

**Claims**

1. Method for detecting spurious signals caused by movements of a patient or his environment in the pulse oximetric measurement of the arterial oxygen saturation, in which the intensity of the light emerging from the test specimen is measured by two or more photodiodes (7, 8), the signals ($S_1$, $S_2$) generated at these photodiodes (7, 8) are normalized to equal levels of their DC components and, to detect spurious signals caused by movements, the differences ($\Delta S_n$) of these normalized signals ($S_{1n}$, $S_{2n}$) are formed, the amplitudes of the difference signals ($\Delta S_n$) obtained in the difference formation being used as a measure of the magnitude of the spurious signals caused by movements.

2. Method according to Claim 1, **characterized in that** the ratio between the amplitude of a difference signal ($\Delta S_n$) and the amplitude of a normalized signal ($S_{1n}$, $S_{2n}$) is formed and this ratio is used as a measure of the relevance of the spurious signals caused by movements with respect to their influence on the precision of the pulse oximetric measurement of the arterial oxygen saturation.

3. Method according to Claim 1 or 2, **characterized in that** the normalization of the signals ($S_1$, $S_2$) generated at the photodiodes (7, 8) takes place in that in a first step these are logarithmized and in a second step the DC components of the logarithmized signals are removed with the aid of high pass filters.

**Revendications**

1. Procédé de détection des signaux parasites générés par les mouvements d'un patient ou de son environnement lors de la mesure pulsoxymétrique de la saturation en oxygène artérielle, dans lequel on mesure à l'aide de deux photodiodes (7, 8) ou plus, l'intensité de la lumière sortant de l'objet mesuré, on normalise les signaux ($S_1$, $S_2$) produits par les photodiodes (7, 8) à des niveaux de courant continu identiques et, pour détecter les signaux parasites dus à des mouvements, on forme les différence de ces signaux normalisés ($S_{1n}$, $S_{2n}$), les amplitudes des signaux différentiels ($\Delta S_n$) provenant du calcul de la différence étant utilisées comme mesure pour la valeur des signaux parasites dus aux mouvements.

2. Procédé selon la revendication 1, **caractérisé en ce qu'**on forme le rapport de l'amplitude d'un signal différentiel ($\Delta S_n$) à l'amplitude d'un signal normalisé ($S_{1n}$, $S_{2n}$) et on utilise ce rapport en tant que mesure pour le poids des signaux parasites dus à des mouvements sur le plan de leur influence sur la précision de la mesure pulsoxymétrique de la saturation en oxygène artérielle.

3. Procédé selon la revendication 1 ou 2, **caractérisé en ce qu'**on réalise la normalisation des signaux ($S_1$, $S_2$) délivrés par les photodiodes (7, 8) en prenant leur logarithme dans une première étape et en éliminant les fractions de courant continue des signaux logarithmiques à l'aide de filtres passe-haut, dans une seconde étape.

FIG. 1B

FIG. 1A

FIG. 1C

FIG. 2

FIG. 3